# EUROPEAN PATENT APPLICATION

(11) **EP 4 527 338 A1**
(43) Date of publication of application: **26.03.2025**
(21) Application number: 24172534.0
(22) Date of filing: 25.04.2024
(51) Int. Cl.: A61B 34/30, A61B 90/50, A61B 90/57, A61B 17/00

(54) **SURGICAL INSTRUMENTS HOLDING APPARATUS**

(30) Priority: 19.09.2023 KR 20230124534; 19.09.2023 KR 20230124538
(71) Applicant: ROEN Surgical, Inc., Daejeon 34051 (KR)
(72) Inventor: YANG, Un-Je, Gyeonggi-do, 14232 (KR); KONG, Duk-Yoo, Seoul, 06221 (KR); KIM, Duk-Sang, Seoul, 06989 (KR); BAEK, Hyunwoo, Seoul, 06151 (KR); KIM, Joonyeong, Gyeonggi-do, 16690 (KR)
(74) Representative: Stolmár & Partner Patentanwälte PartG mbB

(57) **Abstract**

Provided is a surgical instrument fixing device including: a first body to which a surgical instrument is fixed; and a second body which is spaced apart from or in close contact with the first body and to which the surgical instrument is fixed. Here, the first body includes a first body part including a first accommodating part in which the surgical instrument is accommodated and a first fastening part including a plurality of protrusions protruding toward the second body. Also, the second body includes a second body part including a second accommodating part in which the surgical instrument is accommodated and a second fastening part including a plurality of grooves recessed from the second body part toward the first body. Also, each of the plurality of protrusions and the plurality of grooves are arranged along a center of the first or second fastening part.

## Description

### BACKGROUND

The present invention relates to a surgical instrument fixing device, and more particularly, to a surgical instrument fixing device capable of fixing a surgical instrument at an exact position.

A surgical operation using a robot is introduced for perfection of surgery. Since a position of a surgical instrument may be changed more precisely when a robot is used than when a person directly performs surgery, the surgery may be performed more successfully.

The surgical instrument is required to be accurately fixed to a set position for successful surgery. Here, a process of aligning the surgical instrument to the set position and a process of fixing the surgical instrument to the set position are important.

When the position of the surgical instrument is not aligned or fixed to the set position, the surgery may produce a different result. Even when a micrometer-level error occurs because the surgical robot is not fixed to the set position, the surgery may produce a different result.

To this end, a device capable of fixing the surgical instrument to an exact position is being researched.

### [RELATED ART DOCUMENTS]

### [PATENT DOCUMENTS]

Korean Patent Registration No. 10-1569995 (published on November 30, 2015)

### SUMMARY

The present invention provides a surgical instrument fixing device capable of aligning a surgical instrument to an exact position and fixing the surgical instrument to prevent the surgical instrument from being deviated from the aligned position.

The present invention also provides a surgical instrument fixing device capable of firmly coupling bodies that fix a surgical instrument to each other.

The present invention also provides a surgical instrument fixing device allowing bodies that fix a surgical instrument to be easily coupled to each other.

The present invention also provides a surgical instrument fixing device capable of easily eliminating an error generated when bodies that fix a surgical instrument are coupled to each other.

An embodiment of the present invention provides a surgical instrument fixing device including: a first body to which a surgical instrument is fixed; a second body which is spaced apart from or in close contact with the first body and to which the surgical instrument is fixed. Here, the first body includes a first body part including a first accommodating part in which the surgical instrument is accommodated and a first fastening part including a plurality of protrusions protruding toward the second body. Also, the second body includes a second body part including a second accommodating part in which the surgical instrument is accommodated and a second fastening part including a plurality of grooves recessed from the second body part toward the first body. Also, each of the plurality of protrusions and the plurality of grooves are arranged along a center of the first or second fastening part.

In an embodiment, angles between a pair of adjacent protrusions among the plurality of protrusions and the center of the first fastening part may be equal to each other, angles between a pair of adjacent grooves among the plurality of grooves and the center of the second fastening part may be equal to each other, the number of the plurality of grooves may be equal to or greater than that of the protrusions, and a volume of each of the grooves may be equal to or greater than that of each of the protrusions.

In an embodiment, the second fastening part may include a first area in which the grooves are defined and a second area surrounding the first area, and the first area may have a surface area equal to or greater than that of the second area on a plane.

In an embodiment, the second fastening part may include a curved surface defined in an area between the first area and the second area.

In an embodiment, each of the protrusions may include a first protruding portion extending in a direction from the first fastening part to the second fastening part and having a cross-sectional area gradually decreasing in a direction toward the second fastening part and a second protruding portion which is disposed on an end of the first protruding portion and in which at least a portion of a surface is curved. Also, each of the grooves may include a first groove portion extending in a direction from the second fastening part to the first fastening part and having a cross-sectional area gradually decreasing in a direction away from the first fastening part and a second groove portion which is disposed on an end of the first groove portion and in which at least a portion of a surface is curved. Also, the curved portion of the second protruding portion and the curved portion of the second groove portion may have curved shapes corresponding to each other.

In an embodiment, on the plane, each of the second protruding portion and the second groove portion may have a polygonal shape with a rounded corner, and the second protruding portion may be fitted and coupled to the second groove portion.

In an embodiment, on the plane, each of the second protruding portion and the second groove portion may have an isosceles triangle shape in which corners are rounded, and a pair of adjacent sides have the same length with an angle between vertices adjacent to a center of the first fastening part and a center of the second fastening part, and six or more groove portions may be provided.

In an embodiment, the first fastening part may include a first support part supporting the protrusions and a second support part supporting the first support part to the first body part, and the second support part may further include a spring member that elastically supports the first support part.

In an embodiment, each of the first fastening part and the second fastening part may be provided in plurality, the first fastening parts may be arranged along the first accommodating part, and the second fastening parts may be arranged along the second accommodating part.

In an embodiment, the first fastening parts may be disposed at the same distance based on the first accommodating part, and the second fastening parts may be disposed at the same distance based on the second accommodating part.

### BRIEF DESCRIPTION OF THE FIGURES

The accompanying drawings are included to provide a further understanding of the inventive concept, and are incorporated in and constitute a part of this specification. The drawings illustrate exemplary embodiments of the inventive concept and, together with the description, serve to explain principles of the inventive concept. In the drawings:
FIG. 1 is a perspective view illustrating a surgical instrument fixing device according to an embodiment of the present invention;
FIG. 2 is a perspective view illustrating a first body of the surgical instrument fixing device according to an embodiment of the present invention;
FIG. 3 is a perspective view illustrating a second body of the surgical instrument fixing device according to an embodiment of the present invention;
FIG. 4 is a plan view illustrating the first body of the surgical instrument fixing device according to an embodiment of the present invention;
FIG. 5 is a plan view illustrating the second body of the surgical instrument fixing device according to an embodiment of the present invention;
FIG. 6 is a perspective view illustrating a first fastening part of the surgical instrument fixing device according to an embodiment of the present invention;
FIG. 7 is a perspective view illustrating a second fastening part of the surgical instrument fixing device according to an embodiment of the present invention;
FIG. 8 is a perspective view illustrating the first fastening part of the surgical instrument fixing device according to an embodiment of the present invention;
FIG. 9 is a perspective view illustrating the first body of the surgical instrument fixing device according to an embodiment of the present invention;
FIGS. 10 and 11 are perspective views illustrating the second body of the surgical instrument fixing device according to an embodiment of the present invention;
FIGS. 12 to 14 are perspective views illustrating a coupled state of the first body and the second body of the surgical instrument fixing device according to an embodiment of the present invention;
FIG. 15 is a perspective view illustrating a surgical instrument fixing device according to another embodiment of the present invention;
FIG. 16 is an exploded view illustrating the surgical instrument fixing device according to another embodiment of the present invention;
FIG. 17 is an enlarged view illustrating a portion of the surgical instrument fixing device according to another embodiment of the present invention;
FIG. 18 is a perspective view illustrating a surgical instrument fixing device according to another embodiment of the present invention;
FIG. 19 is a plan view illustrating the surgical instrument fixing device according to another embodiment of the present invention;
FIG. 20 is a perspective view illustrating the surgical instrument fixing device according to another embodiment of the present invention;
FIG. 21 is a schematic cross-sectional view illustrating the surgical instrument fixing device according to another embodiment of the present invention;

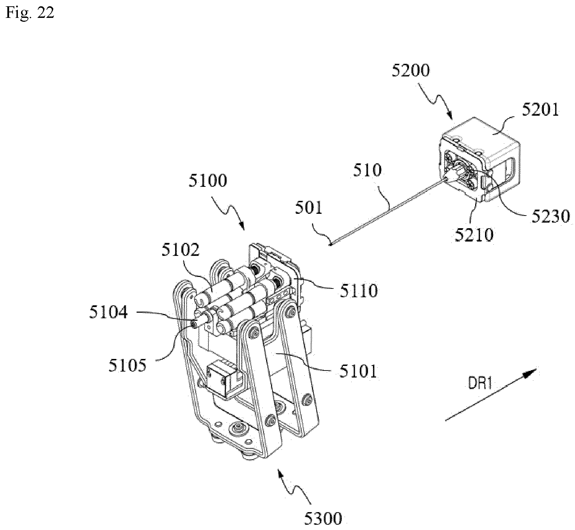

FIG. 22 is an exploded perspective view illustrating the surgical device including an arm module according to the present invention;

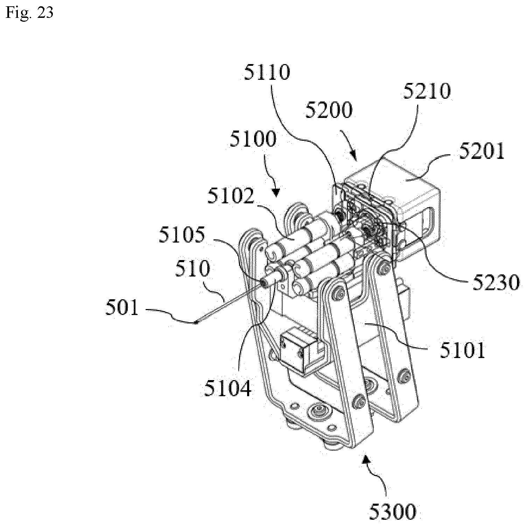

FIG. 23 is an assembled perspective view illustrating the surgical device including the arm module according to the present invention;

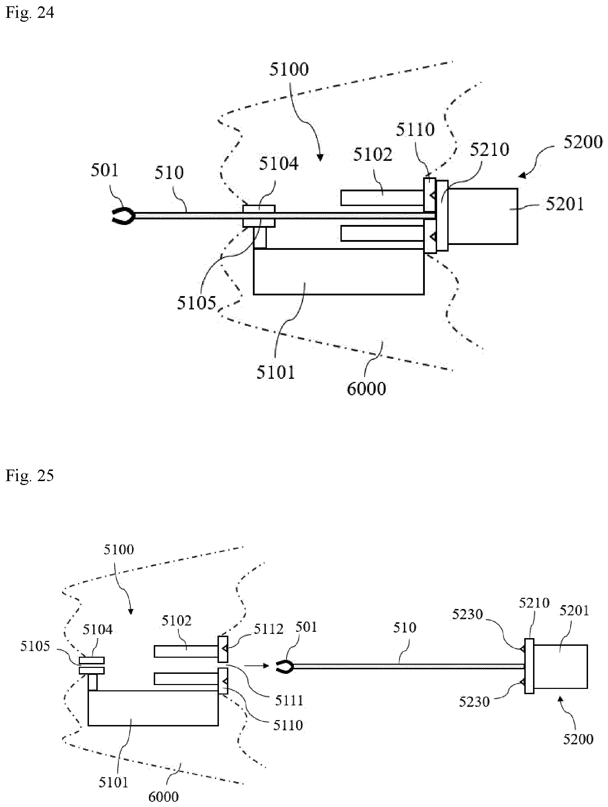

FIG. 24 is a schematic side view of FIG. 23; and
FIG. 25 is a schematic side view of FIG. 22.

### DETAILED DESCRIPTION

In the specification, when it is described that an element (region, layer, or portion) is "coupled to", "disposed on", or "connected to" another element, it should be understood that the element may be directly disposed/coupled/connected to the another element or a third element may be disposed therebetween.

Like reference numerals refer to like elements throughout. Also, in the figures, the thickness, ratio, and dimensions of components are exaggerated for clarity of illustration. The term "and/or" includes any and all combinations of one or more of the associated listed items.

It will be understood that although the terms first and second are used herein to describe various elements, these elements should not be limited by these terms. These terms are only used to distinguish one element from another element. Therefore, a member, a component, a region, a layer, or a portion referred to as a first member, a first component, a first region, a first layer, or a first portion in an embodiment can be referred to as a second member, a second component, a second region, a second layer, or a second portion in another embodiment. The terms of a singular form may include plural forms unless referred to the contrary.

Also, spatially relative terms, such as "below", "lower", "above", and "upper", may be used herein for ease of description to describe an element and/or a feature's relationship to another element(s) and/or feature(s) as illustrated in the drawings. The terms may be a relative concept and described based on directions expressed in the drawings.

The meaning of "include," "comprise," "including," or "comprising," specifies a property, a region, a fixed number, a step, a process, an element and/or a component but does not exclude other properties, regions, fixed numbers, steps, processes, elements and/or components.

Unless otherwise specifically defined herein, all terms including technical or scientific terms are to be given meanings understood by those skilled in the art. Also, like terms defined in dictionaries, generally used terms needs to be construed as meaning used in technical contexts and are not construed as ideal or excessively formal meanings unless otherwise clearly defined herein.

Hereinafter, embodiments of the present invention will be described with reference to the accompanying drawings.

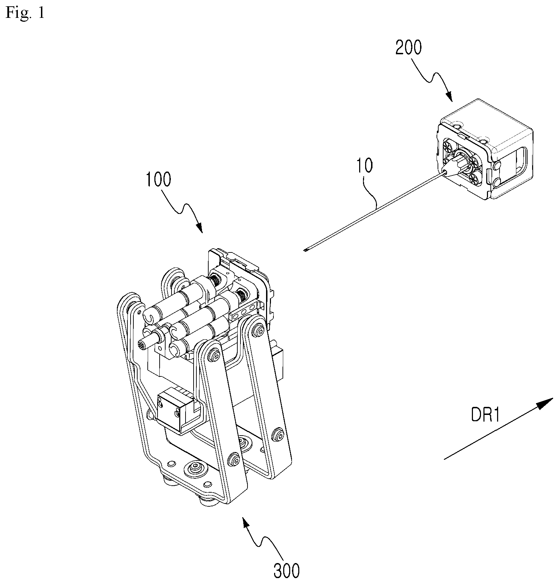

FIG. 1 is a perspective view illustrating a surgical instrument fixing device 30 according to an embodiment of the present invention.

Referring to FIG. 1, the surgical instrument fixing device 30 may include a first body 100, a second body 200, and a drive unit 300.

A surgical instrument 10 may be fixed to the first body 100 and the second body 200. The surgical instrument 10 may be aligned to a position and then prevented from deviating from the aligned position by fixing the surgical instrument 10 to each of the first body 100 and the second body 200.

The second body 200 may be spaced apart from or come into close contact with the first body 100. The drive unit 300 may allow the first body 100 and the second body 200 to be in close contact with or spaced apart from each other.

The drive unit 300 may include an actuator such as a motor. However, the embodiment of the present invention is not limited thereto. For example, the drive unit 300 and may include a device for generating various driving forces.

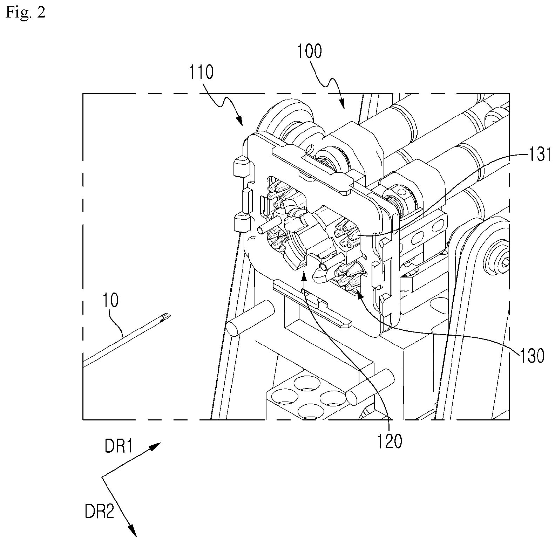

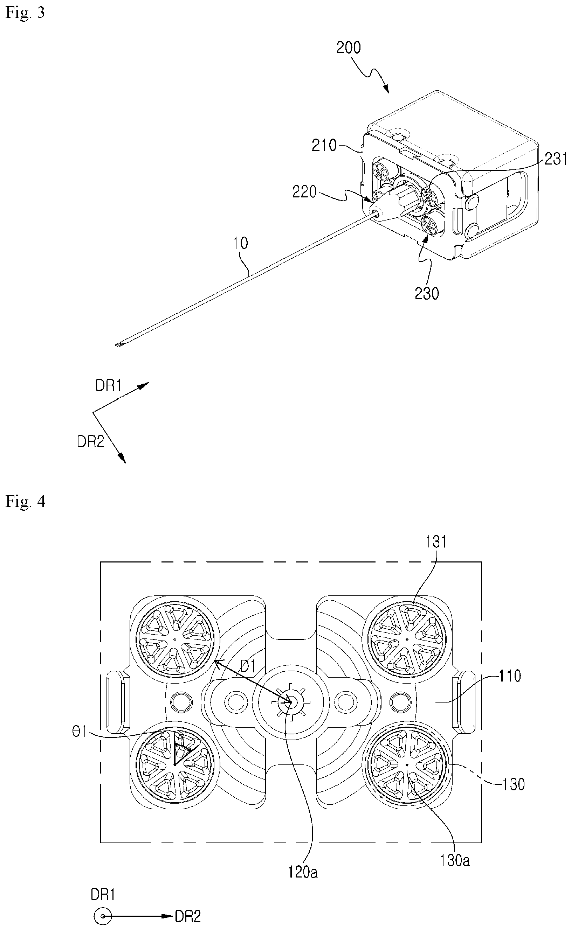

FIG. 2 is a perspective view illustrating the first body 100 of the surgical instrument fixing device 30 according to an embodiment of the present invention. FIG. 3 is a perspective view illustrating the second body 200 of the surgical instrument fixing device 30 according to an embodiment of the present invention.

Referring to FIGS. 2 and 3, the first body 100 may include a first body part 110, a first accommodating part 120, and a first fastening part 130. The second body 200 may include a second body part 210, a second accommodating part 220, and a second fastening part 230.

A first accommodating part 120 in which the surgical instrument 10 is accommodated may be defined in the first body part 110, and a second accommodating part 120 in which the surgical instrument 10 is accommodated may be defined in the second body part 210. As illustrated in the drawing, the first accommodating part 120 and the second accommodating part 220 may have different shapes. When the first body 100 and the second body 200 are coupled to each other, the second accommodating part 220 may be accommodated in the first accommodating part 120.

As illustrated in the drawing, the first accommodating part 120 may have a recessed shape, and the second accommodating part 120 may have a protruding shape. An opening through which the surgical instrument 10 passes may be defined in each of the first accommodating part 120 and the second accommodating parts 220.

According to an embodiment of the present invention, a first state in which the first body 100 and the second body 200 are coupled to each other and a second state in which the coupled state of the first body 100 and the second body 200 is released may be defined. In the first state, the first fastening part 130 of the first body 100 and the second fastening part 230 of the second body 200 are fastened to each other, and in the second state, the fastening state of the first fastening part 130 of the first body 100 and the second fastening part 230 of the second body 200 is released.

The first fastening part 130 may protrude from the first body part 110 toward the second body 200. The second fastening part 230 may protrude from the second body part 210 toward the first body 100.

Also, the first fastening part 130 may include a plurality of protrusions 131 protruding from the first body part 110 toward the second body 200, and the second fastening part 230 may include a plurality of grooves 231 recessed from the second body part 210 toward the first body 100. The protrusions 131 and grooves 231 may be accommodated in the corresponding grooves 231 and protrusions 131, respectively, and may be fitted and coupled to each other as an example.

As the protrusions 131 are accommodated in the grooves 231, respectively, the first body 100 and the second body 200 may be firmly coupled to prevent the surgical instrument 10 from being deviated from the aligned position.

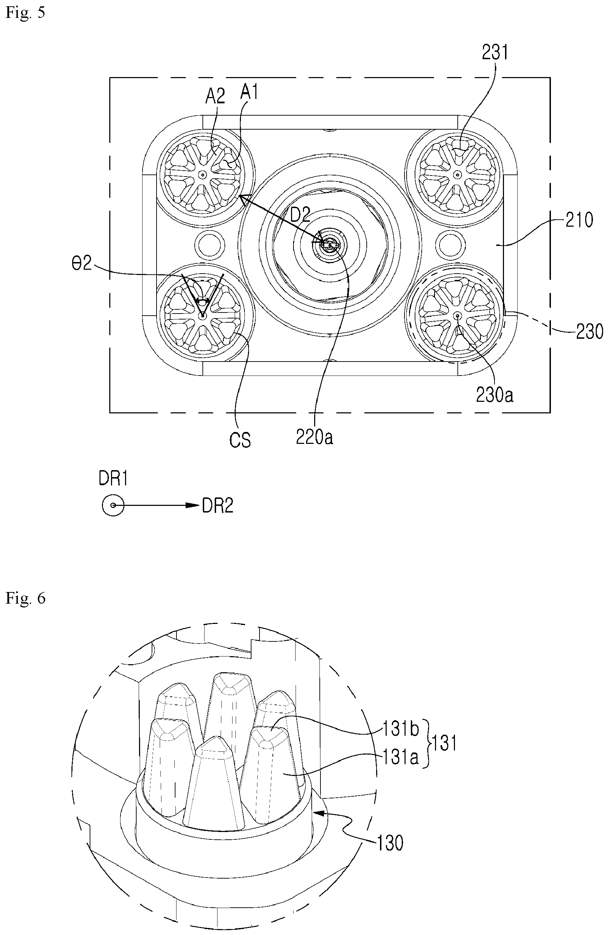

FIG. 4 is a plan view illustrating the first body 100 of the surgical instrument fixing device 30 according to an embodiment of the present invention. FIG. 5 is a plan view illustrating the second body 200 of the surgical instrument fixing device 30 according to an embodiment of the present invention.

Referring to FIGS. 4 and 5, each of the first fastening part 130 and the second fastening part 230 may be provided in plurality. The number of first fastening parts 130 and the number of second fastening parts 230 may correspond to each other.

The plurality of first fastening parts 130 may be arranged along a center 120a of the first accommodating part 120. The plurality of second fastening parts 230 may be arranged along a center 220a of the second accommodating part 220. A distance D1 between each of the plurality of first fastening parts 130 and the center 120a of the first accommodating part 120 may be the same. A distance D2 between each of the plurality of second fastening parts 230 and the center 220a of the second accommodating part 220 may be the same. Here, the distance D1 between the center 120a of the first accommodating part 120 and the first fastening part 130 may be equal to the distance D2 between the center 220a of the second accommodating part 220 and the second fastening part 230.

According to an embodiment of the present invention, the plurality of protrusions 131 may be arranged along a center of the first fastening part 130. According to an embodiment of the present invention, a distance between each of the plurality of protrusions 131 and a center 130a of the first fastening part 130 may be the same. An angle θ1 between a pair of adjacent protrusions 131 and the center 130a of the first fastening portion 130 may be the same.

As described above, the first body 100 and the second body 200 may be easily coupled to each other in the first state by including the plurality of protrusions 131 and having the same angle θ1 between the pair of protrusions 131.

The plurality of grooves 231 may be arranged along the center 230a of the second fastening part 230. According to an embodiment of the present invention, a distance between each of the plurality of grooves 231 and the center 230a of the second fastening part 230 may be the same. An angle θ2 between a pair of adjacent grooves 231 and the center 230a of the second fastening part 230 may be the same. According to an embodiment, the angle θ1 between the above-described adjacent protrusions 131 and the center of the first fastening part 130 may be equal to the angle θ2 between the corresponding adjacent grooves 231 and the center of the second fastening part 230.

According to an embodiment of the present invention, the number of grooves 231 may be equal to or greater than that of protrusions 131. Through this, the protrusions 131 may be easily accommodated in the grooves 231. In this case, a planar shape of each of the grooves 231 may be the same as that of each of the protrusions 131. However, the embodiment of the present invention is not limited thereto.

According to an embodiment of the present invention, six or more grooves 231 may be provided. Here, the number of protrusions 131 may be equal to or less than that of grooves 231. However, the embodiment of the present invention is not limited thereto.

On a plane, each of the protrusions 131 and grooves 231 may have a polygonal shape with rounded corners. For example, each of the protrusions 131 and the grooves 231 may have a shape such as an isosceles triangle, an equilateral triangle, and a trapezoid with rounded corners. However, the embodiment of the present invention is not limited thereto.

According to an embodiment of the present invention, the second fastening part 230 may include a first area A1 and a second area A2 surrounding the first area A1. The grooves 231 are defined in the first area A1, and the second area A2 surrounds the grooves 231 and protrudes relative to the grooves 231. That is, the second area A2 may be closer to the first body part 110 than the first area A1.

On the plane, the first area A1 may have a surface area equal to or greater than that of the second area A2. Through this, the protrusions 131 may be easily accommodated in the grooves 231.

A curved surface CS may be defined between the first area A1 and the second area A2. Although the protrusion 131 may be directly accommodated in the groove 231, the protrusion 131 may be also inserted into the groove 231 through the curved surface CS defined in the first area A1 and the second area A2, which allows the bodies 100 and 200 to be easily coupled to each other.

Each of the protrusions 131 may have the same volume as each of the corresponding grooves 231. The protrusions 131 and the grooves 231 may be fitted and coupled to each other due to the same volume. Through this, after the protrusions 131 and the grooves 231 are coupled to each other, the protrusions 131 are prevented from being deviated from positions thereof in the grooves 231 to prevent the aligned position of the surgical instrument 10 from being varied.

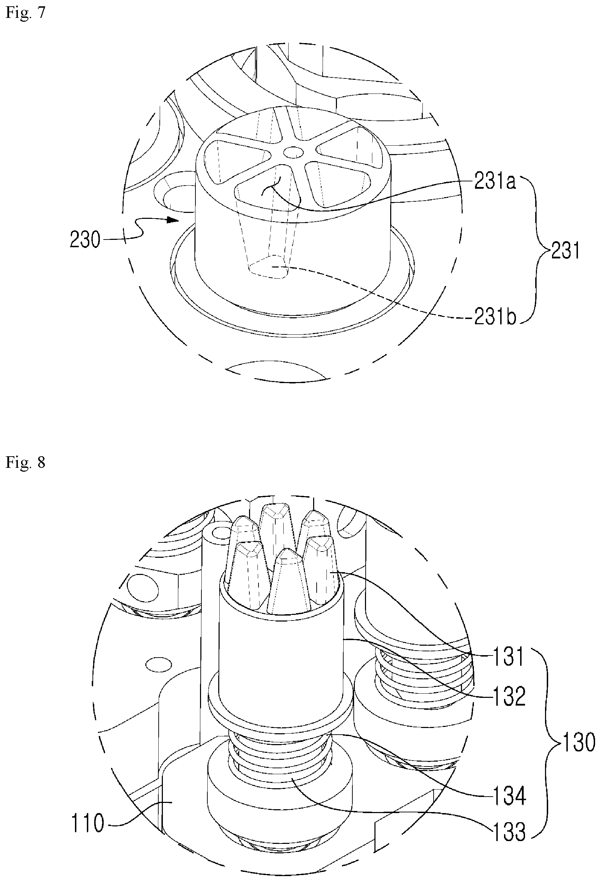

FIG. 6 is a perspective view illustrating the first fastening part 130 of the surgical instrument fixing device 30 according to an embodiment of the present invention. FIG. 7 is a perspective view illustrating the second fastening part 230 of the surgical instrument fixing device 30 according to an embodiment of the present invention.

Referring to FIGS. 6 and 7, the protrusion 131 may include a first protruding portion 131a and a second protruding portion 131b.

The first protruding portion 131a extends in a direction from the first fastening part 130 to the second fastening part 230. The protrusion 131 may have a tapered shape. Specifically, a cross-sectional area of the first protruding portion 131a in a second direction DR2 crossing the first direction DR1 may gradually decrease in a direction toward the second fastening part 230.

The second protruding portion 131b may be disposed at an end of the first protruding portion 131a, and at least a portion of a surface thereof may be curved.

The groove 231 may include a first groove portion 231a and a second groove portion 231b. The first groove portion 231a may extend in a direction away from the first fastening part 130. The groove 231 may have a tapered shape. Specifically, a cross-sectional area of the first groove portion 231a in the second direction DR2 may gradually decrease in a direction away from the second fastening part 230.

The second protruding portion 231b may be disposed at an end of the first groove portion 231a, and at least a portion of a surface thereof may be curved.

Each of the protrusion 131 and the groove 231 may have a tapered shape, and at least a portion of the end may be curved so that the protrusion 131 is easily accommodated in the groove 231. Through this, the first body 100 and the second body 200 may be easily coupled to each other.

The first protruding portion 131a and the first groove portion 231a may have shapes corresponding to each other, and the second protruding portion 131b and the second groove portion 231b may have shapes corresponding to each other. For example, a curved portion of the second protruding portion 131b and a curved portion of the second groove portion 231b may have shapes corresponding to each other. Through this, the protrusion 131 and the groove 231 may be easily fitted and coupled to each other.

On the plane, each of the second protruding portion and the second groove portion 231b may have a polygonal shape with rounded corners. Specifically, the second protruding portion and the second groove portion 231b may each have a polygonal shape with rounded corners based on a cross-section parallel to the second direction DR2.

According to an embodiment of the present invention, the second protruding portion 131b may have an isosceles triangle shape in which corners are rounded, and a pair of adjacent sides have the same length with an angle between vertices adjacent to the center 130a of the first fastening part 130. However, the embodiment of the present invention is not limited thereto. For example, the second protruding portion 131b may have an equilateral triangle shape or a fan shape.

The second groove portion 231b may have an isosceles triangle shape in which corners are rounded, and a pair of adjacent sides have the same length with an angle between vertices adjacent to the center 230a of the second fastening part 230. However, the embodiment of the present invention is not limited thereto. For example, the second groove portion 231b may have an equilateral triangle shape or a fan shape.

Here, the second protruding portion 131b and the second groove portion 231b may have shapes corresponding to each other. In other words, the second protruding portion 131b and the second groove portion 231b may have substantially the same shape.

Through this, although the first body 100 or the second body 200 rotates at an angle smaller than the angle therebetween, the protrusion 131 that is not accommodated in the groove 231 may be accommodated in the groove 231, and thus the first body 100 and the second body 200 may be easily coupled to each other.

FIG. 8 is a perspective view illustrating the first fastening part 130 of the surgical instrument fixing device 30 according to an embodiment of the present invention.

Referring to FIG. 8, the first fastening part 130 according to an embodiment of the present invention may include a first support 132, a second support 133, and a spring member 134.

The first support 132 may support the protrusions 131, the second support 133 may support the first support 132 to the first body part 110, and the spring member 134 may elastically support the first support 132.

As illustrated in the drawing, the spring member 134 may surround the second support 133 and elastically support the first support 132 to the second support 133 or the first body part 110.

As the spring member 134 elastically supports the first support 132, when the protrusion 131 comes into close contact with the second area A2 instead of being accommodated in the groove 231, a position of the protrusion 131 may be changed by an elastic force of the spring member 134, and the protrusion 131 may be accommodated in the groove 231. Through this, although an error in which the protrusion 131 is not accommodated in the groove 231 when the first body 100 and the second body 200 are coupled occurs, the error may be easily removed by using the elastic force of the spring member 134, and the protrusion 131 may be accommodated in the groove 231.

According to an embodiment, a length of the second support 133 may be varied together when a length of the spring member 134 is varied according to pressure applied to the first support 132.

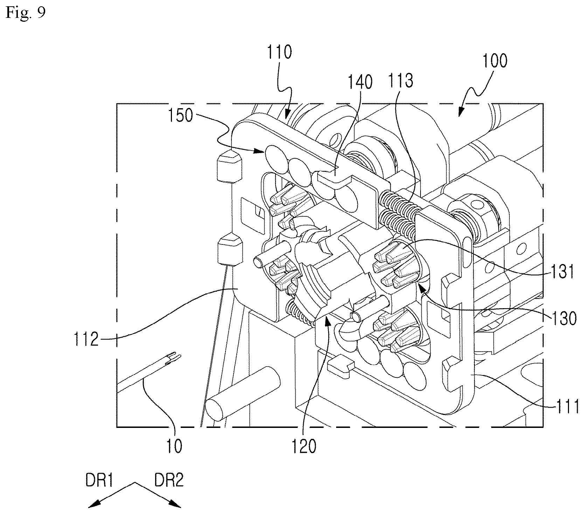

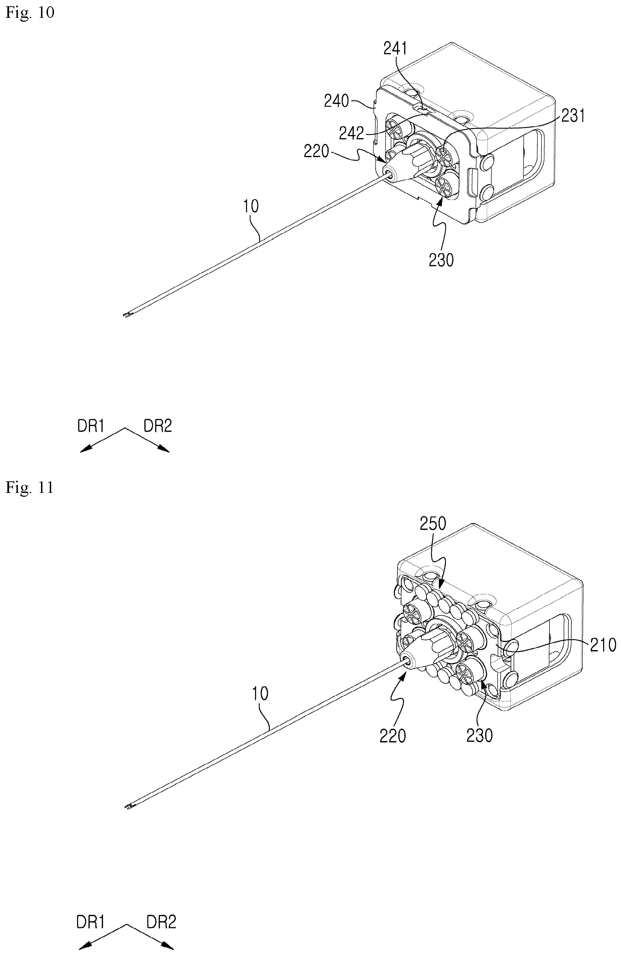

FIG. 9 is a perspective view illustrating the first body 100 of the surgical instrument fixing device 30 according to an embodiment of the present invention. FIGS. 10 and 11 are perspective views illustrating the second body 200 of the surgical instrument fixing device 30 according to an embodiment of the present invention.

Referring to FIGS. 9 to 11, the first body 100 may include a first body part 110, a first accommodating part 120, a first fastening part 130, a first coupling part 140, and a first magnetic part 150. The second body 200 may include a second body part 210, a second accommodating part 220, a second fastening part 230, a second coupling part 240, and a second magnetic part 250.

A first accommodating part 120 in which the surgical instrument 10 is accommodated may be defined in the first body part 110, and a second accommodating part 120 in which the surgical instrument 10 is accommodated may be defined in the second body part 210. As illustrated in the drawing, the first accommodating part 120 and the second accommodating part 220 may have different shapes. When the first body 100 and the second body 200 are coupled to each other, the second accommodating part 220 may be accommodated in the first accommodating part 120.

As illustrated in the drawing, the first accommodating part 120 may have a recessed shape, and the second accommodating part 120 may have a protruding shape. An opening through which the surgical instrument 10 passes may be defined in each of the first accommodating part 120 and the second accommodating parts 220.

According to an embodiment of the present invention, a first state in which the first body 100 and the second body 200 are coupled to each other and a second state in which the coupled state of the first body 100 and the second body 200 is released may be defined. In the first state, the first fastening part 130 of the first body 100 and the second fastening part 230 of the second body 200 are fastened to each other, and in the second state, the fastening state of the first fastening part 130 of the first body 100 and the second fastening part 230 of the second body 200 is released.

The first fastening part 130 may protrude from the first body part 110 toward the second body 200. The second fastening part 230 may protrude from the second body part 210 toward the first body 100.

Also, the first fastening part 130 may include a plurality of protrusions 131 protruding from the first body part 110 toward the second body 200, and the second fastening part 230 may include a plurality of grooves 231 recessed from the second body part 210 toward the first body 100. The protrusions 131 and grooves 231 may be accommodated in the corresponding grooves 231 and protrusions 131, respectively, and may be fitted and coupled to each other as an example.

As the protrusions 131 are accommodated in the grooves 231, respectively, the first body 100 and the second body 200 may be firmly coupled to prevent the surgical instrument 10 from being deviated from the aligned position.

The first coupling part 140 is disposed on the first body part 110, and the second coupling part 240 is disposed on the second body part 210. The first coupling part 140 and the second coupling part 240 may be coupled to or released from each other.

The first coupling part 140 of the first body 100 and the second coupling part 240 of the second body 200 are coupled to each other in the first state, and the coupled state of the first coupling part 140 and the second coupling part 240 is released. Specifically, a ratchet of the first coupling part 140 may be accommodated in a ratchet groove 241 defined in the second coupling part 240.

The first magnetic part 150 may be disposed on the first body part 110 , and the second magnetic part 250 may be disposed on the second body part 120. Depending on a relative position of the first magnetic part 150 and/or the second magnetic part 250, an attractive or repulsive force may act between the first magnetic part 150 and the second magnetic part 250.

In the first state, an attractive force may act between the first magnetic part 150 of the first body 100 and the second magnetic part 250 of the second body 200. In the first state, the attractive force between the first magnetic part 150 and the second magnetic part 250 may strengthen a coupling relationship between the first coupling part 140 and the second coupling part 240, fix positions of the first body 100 and the second body 200, and prevent the surgical instrument 10 from being deviated from the aligned position. In the second state, a repulsive force acts between the first magnetic part 150 and the second magnetic part 250. In the second state, the repulsive force between the first magnetic part 150 and the second magnetic part 250 may release a position fixed state of the first body 100 and the second body 200, release the coupled state by weakening a fixing force between the first coupling part 140 and the second coupling part 240, and allow the first body 100 and the second body 200 to be spaced apart from each other.

Specifically, as the second magnetic part 250 is disposed at a position corresponding to the first magnetic portion 150 in the first state, the attractive force may act. In the second state, as the positions of the first magnetic part 150 or the second magnetic part 250 are partially changed, the positions may be changed from those of the first state. Due to this, the repulsive force may act between the first magnetic part 150 and the second magnetic part 250.

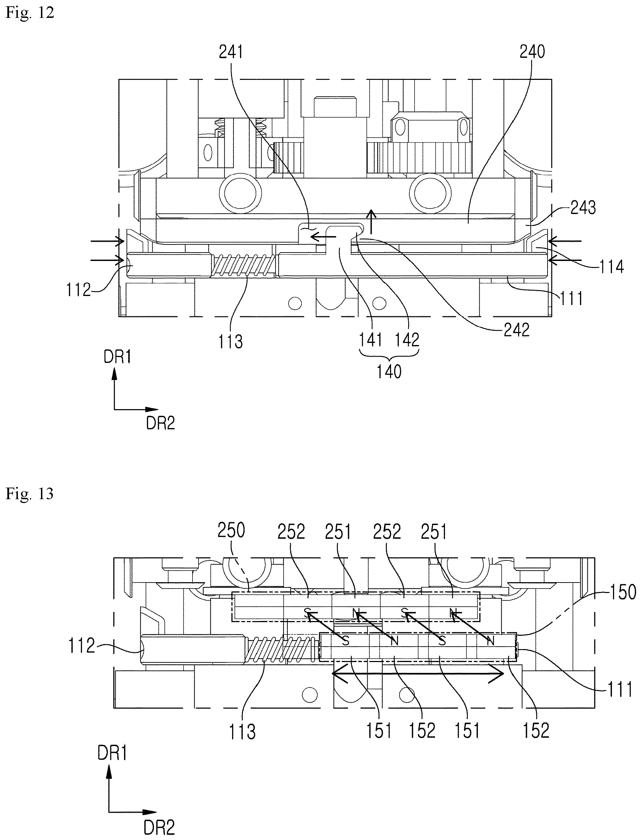

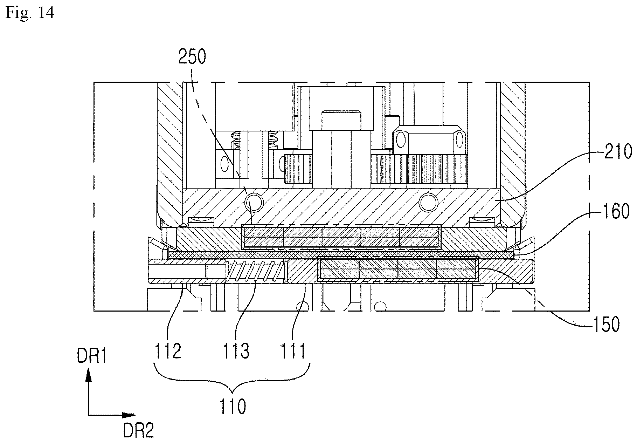

FIGS. 12 to 14 are perspective views illustrating the coupled state of the first body and the second body of the surgical instrument fixing device according to an embodiment of the present invention.

The ratchet may include a first part 141 protruding from the first body part 110 toward the second body 200 and a second part 142 bent from the first part 141. A ratchet groove 241 capable of accommodating the ratchet is defined in the second coupling part 240, and a step 242 capable of supporting the second part 142 accommodated in the ratchet groove 241 may be formed.

As the ratchet is accommodated in the ratchet groove 241, and the second part 142 of the ratchet is supported by the step 242, the coupled state of the first coupling part 140 and the second coupling part 240 may be strengthened.

The first body part 110 may include a first frame 111, a second frame 112, and an elastic member 113. The first frame 111 and the second frame 112 may be spaced apart from each other in the second direction DR2 crossing the first direction DR1. The elastic member 113 may be disposed between the first frame 111 and the second frame 112 to elastically support the first frame 111 and the second frame 112.

The first frame 111 and the second frame 112 may be relatively moved in the second direction DR2 by applying an external force from the outside. For example, the first frame 11 and the second frame 112 may be relatively moved in the second direction DR2 by using an actuator instead of applying the external force. However, the embodiment of the present invention is not limited thereto.

The first frame 111 and the second frame 112 may be relatively moved in the second direction DR2 by arranging an elastic member 113 between the first frame 111 and the second frame 112. That is, in a process of converting from the first state to the second state, the first frame 111 and the second frame 112 may be relatively moved in the second direction DR2, and the elastic member 113 that elastically supports the first frame 111 and the second frame 112 may be varied in length in the second direction DR2.

While the first frame 111 and the second frame 112 are relatively moved in the second direction DR2, the ratchet may be accommodated in the ratchet groove 241 and then released from the accommodated state.

According to an embodiment of the present invention, each of the first magnetic part 150 and the second magnetic part 250 may include a plurality of magnetic members. The first magnetic part 150 may include a plurality of first magnetic members 151 and a plurality of second magnetic members 152, and the first magnetic member 151 and the second magnetic member 152 may have different magnetism. The second magnetic part 250 may include a plurality of third magnetic members 251 and a plurality of fourth magnetic members 252, and the third magnetic member 251 and the fourth magnetic member 252 may have different magnetism.

Here, the magnetic member may be a permanent magnet. However, the embodiment of the present invention is limited thereto. For example, the magnetic member may be an electromagnet, or a portion of the magnetic member may be a permanent magnet and a portion thereof is an electromagnet.

The first magnetic member 151 and the third magnetic member 251 may have different magnetism, and the second magnetic member 152 and the fourth magnetic member 252 may have different magnetism.

In the first state, the first magnetic member 151 and the third magnetic member 251 may overlap each other on the plane, and the second magnetic member 152 and the fourth magnetic member 252 may overlap each other on the plane. As an attractive force occurs between the first magnetic member 151 and the third magnetic member 251 and between the second magnetic member 152 and the fourth magnetic member 252, positions of the first body 100 and the second body 200 may be fixed.

In the process of converting from the first state to the second state, the first magnetic member 151 and the fourth magnetic member 252 may overlap each other on the plane, and the second magnetic member 152 an the third magnetic member 251 may overlap each other on the plane. A repulsive force may occur between the first magnetic member 151 and the fourth magnetic member 252 and between the second magnetic member 152 and the third magnetic member 251 to release the position fixed state of the first body 100 and the second body 200, and the first body 100 and the second body 200 may be spaced apart from each other.

In this case, the first magnetic member 151 and the third magnetic member 251 may partially overlap each other, and the second magnetic member 152 and the fourth magnetic member 252 may partially overlap each other. However, the embodiment of the present invention is not limited thereto.

According to an embodiment of the present invention, the first magnetic member 151 and the second magnetic member 152 may be arranged alternately in one direction, and the third magnetic member 251 and the fourth magnetic member 252 may be arranged alternately in one direction. Here, the one direction may be the second direction DR2 crossing the first direction.

According to the relative movement of the first frame 111 and the second frame 112, an overlap relationship of the first to fourth magnetic members 151, 152, 251, and 252 may be changed. Specifically, in the first state, the first magnetic member 151 and the third magnetic member 251 may overlap each other, and the second magnetic member 152 and the fourth magnetic member 252 may overlap each other. As the first frame 111 and the second frame 112 are relatively moved in the second direction DR2 in the process of converting from the first state to the second state, the first magnetic member 151 and the fourth magnetic member 252 may overlap each other, and the second magnetic member 152 and the third magnetic member 251 may overlap each other.

In the first state, the first body 100 and the second body 200 may be firmly fixed through a balance of: an elastic support force of the elastic member 113; a fastening force through a fastened state of the ratchet 140 and the ratchet groove 241; an attractive force between the first magnetic member 151 and the third magnetic member 251; and an attractive force between the second magnetic member 152 and the fourth magnetic member 251. Through this, the surgical instrument 10 aligned to a correct position may be maintained in position to provide successful surgery.

In the second state, the fixed state of the first body 100 and the second body 200 may be released, and the first body 100 and the second body 200 may be spaced apart from each other by breaking the balance of: the elastic support force of the elastic member 113; the fastening force through the fastened state of the ratchet 140 and the ratchet groove 241; the attractive force between the first magnetic member 151 and the third magnetic member 251; and the attractive force between the second magnetic member 152 and the fourth magnetic member 251. Also, according to the relative movement of the first magnetic member 151 and the second magnetic member 152, the overlapping relationship of the first to fourth magnetic members 151, 152, 251, and 252 may be changed, and the first body 100 and the second body 200 may be easily spaced apart from each other by using the repulsive force of the first magnetic member 151 and the fourth magnetic member 252 and the repulsive force of the second magnetic member 152 and the third magnetic member 251.

According to an embodiment of the present invention, the first body 100 may include a first inclined portion 114, and the second body 200 may include a second inclined portion 243. Specifically, the first body portion 110 may include the first inclined portion 114 having a first inclined surface that is inclined in a direction crossing the first direction DR1, and the second coupling part 240 may include the second inclined portion 243 having a second inclined surface that is inclined in the direction crossing the first direction DR1 and faces the first inclined surface at a position corresponding to the first inclined portion 114. The first inclined surface may have the same inclined direction as the second inclined surface.

In the process of converting from the first state to the second state, the first inclined portion 114 may be moved along the second inclined surface of the second inclined portion 243 in a state in which the first body 100 and the second body 200 are spaced apart from each other. Alternatively, the second inclined portion 243 may be moved along the first inclined surface of the first inclined portion 114.

As the first body 100 and/or the second body 200 are provided to slide along the inclined surfaces of the first inclined portion 114 and the second inclined portion 243, the first body 100 and the second body 200 may be easily spaced apart from each other in the second state. Alternatively, the state in which the ratchet is accommodated in the ratchet groove 241 may be easily released.

According to an embodiment of the present invention, each of the first magnetic part 150 and the second magnetic part 250 may include a plurality of magnetic members. Some of the first magnetic parts 150 may be disposed on the first frame 111, and the rest may be disposed on the second frame 112. Some of the second magnetic parts 250 may be disposed in correspondence to the first magnetic parts 150 disposed on the first frame 111, and the rest are disposed in correspondence to the first magnetic parts 150 disposed on the second frame 112.

According to an embodiment of the present invention, the first body 100 may include a movement restricting member 160 that restricts movement directions of the magnetic members 151 and 152 of the first magnetic part 150. The movement restricting member 160 may move the first magnetic members 151 and the second magnetic members 152 only in the second direction DR2. That is, the movement restricting member 160 may restrict a movement in a direction different from the second direction DR2 except for the movement in the second direction DR2.

The movement restricting member 160 may be coupled to the first body part 110. However, the embodiment of the present invention is not limited thereto. For example, the movement restricting member 160 may be coupled to the second coupling part 240 or the second body part 210 in the first state.

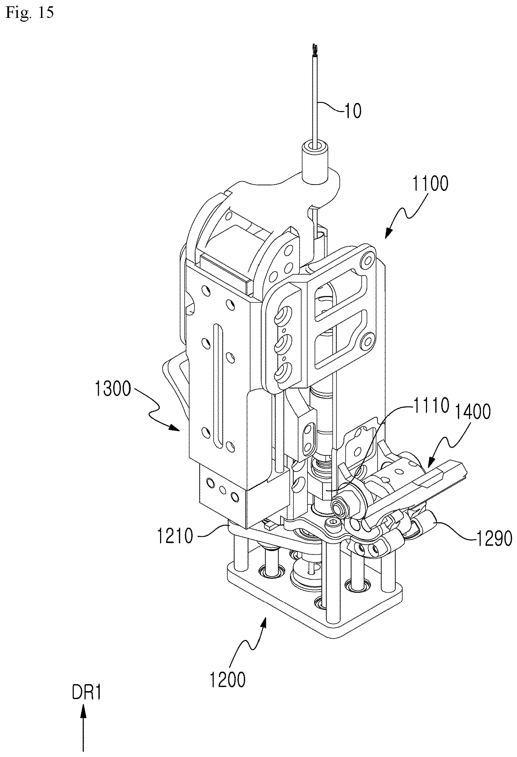

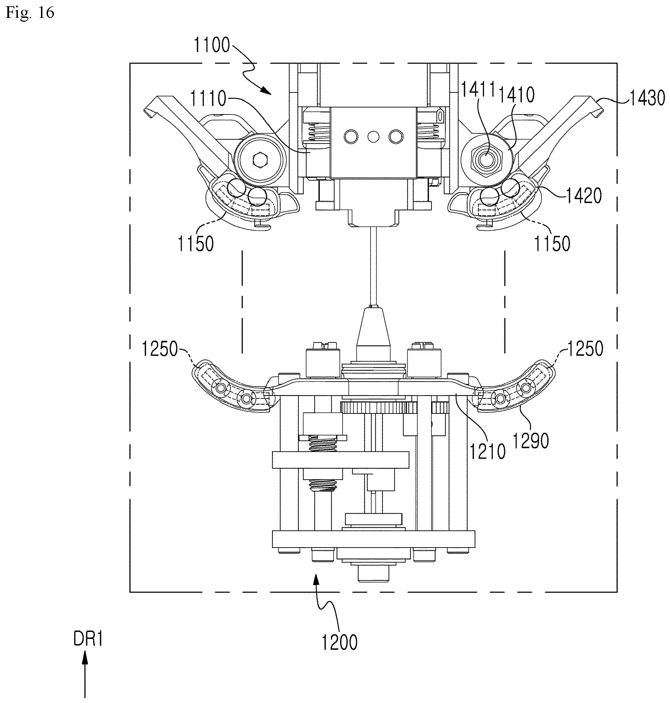

FIG. 15 is a perspective view illustrating a surgical instrument fixing device according to another embodiment of the present invention, and FIG. 16 is an exploded view illustrating the surgical instrument fixing device according to another embodiment of the present invention.

The same or similar components described above in FIGS. 9 to 14 are designated by the same name or reference numerals in which numbers in low place values are matched, and a description thereof will be omitted.

Referring to FIG. 15, according to an embodiment of the present invention, a first body 1100 may include a position adjustment unit 1400 that moves a position of a first magnetic part 1150. The position adjustment unit 1400 may be disposed on a first body part 1110.

The position adjustment unit 1400 may include a first rotation member 1410 and a second rotation member 1420. The first rotation member 1410 may include a rotation shaft 1411 coupled to the first body 1100 and rotating around a direction crossing the first direction DR1. The second rotation member 1420 may rotate around the rotation shaft 1411 disposed on the first rotation member 1410. The first magnetic part 1150 may be coupled to the second rotation member 1420.

The position adjustment unit 1400 may include a wing member 1430. The second rotation member 1420 may rotate by applying an external force to the wing member 1430.

A second body part 1210 of the second body 1200 may include a second magnetic part 1250. The second magnetic part 1250 may be disposed on a support member 1290 of the second body part.

In the first state, a first magnetic member 1151 and a third magnetic member 1251 may come into contact with each other, so that an attractive force acts therebetween, and a second magnetic member 1152 and a fourth magnetic member 1252 may come into contact with each other, so that an attractive force acts therebetween. Through this, the first body 1100 and the second body 1200 may be effectively fixed.

In the process of converting from the first state to the second state, the second rotation member 1420 may rotate around the rotation shaft 1411. As the second rotation member 1420 rotates, the first magnetic member 1151 may come into contact with the fourth magnetic member 1252, and the second magnetic member 1152 may come into contact with the third magnetic member 1251. Accordingly, a repulsive force may act between the first magnetic member 1151 and the third magnetic member 125 and between the second magnetic member 1152 and the fourth magnetic member 1251. Through this, the fixed state of the first body 1100 and the second body 1200 may be released.

The position adjustment unit 1400 may be provided in plurality, and the position adjustment units 1400 may be disposed on at least one of side surfaces of the first body 1100. As illustrated in the drawing, the position adjustment units 1400 may be disposed on the side surfaces of the first body 1100 that face each other. However, the embodiment of the present invention is not limited thereto. For example, the position adjustment units 1400 may be arranged along a circumference of the first body 1100.

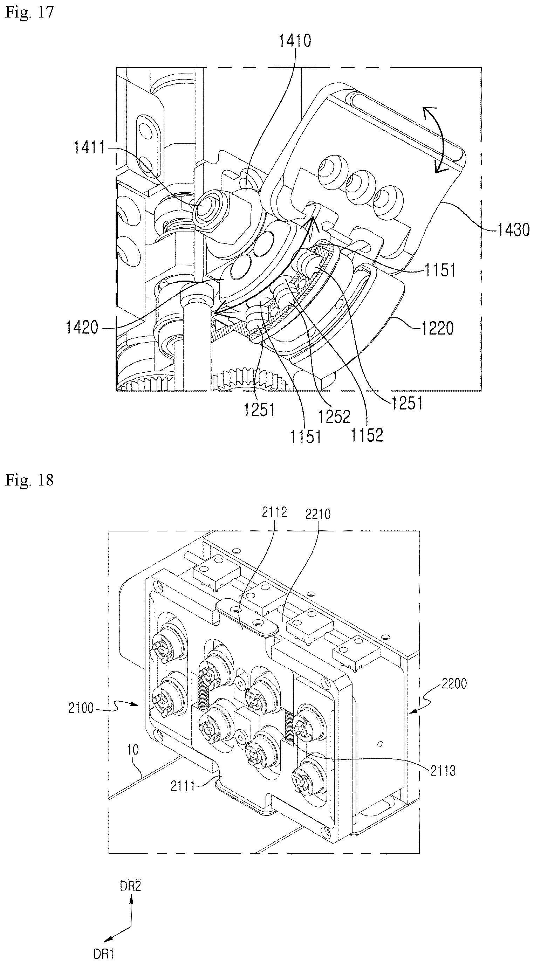

FIG. 17 is an enlarged view illustrating a portion of the surgical instrument fixing device according to another embodiment of the present invention.

Referring to FIG. 17, the first magnetic member 1151 and the second magnetic member 1152 may be arranged alternately in one direction, and the third magnetic member 1251 and the fourth magnetic member 1252 may be arranged alternately in one direction. Here, the one direction may be a circumferential direction based on the rotation shaft 1411.

According to an embodiment of the present invention, the second rotation member 1420 may have a fan shape around the rotation shaft 1411 of the first rotation member 1410, and the first magnetic members 1151 and the second magnetic members 1152 may be disposed on an arc of the fan shape.

The second body 1200 may be connected to the second body part 1210 and have a shape corresponding to the arc of the fan shape, and include a support member 1290 on which the third magnetic members 1251 and the fourth magnetic members 1252 are disposed.

As the support member 1290 has a shape corresponding to the arc of the fan shape of the second rotation member 1420, and the third magnetic members 1251 and the fourth magnetic members 1252 are disposed on positions corresponding to the first magnetic members 1151 and the second magnetic force disposed on the arc of the second rotation member 1420, attractive and repulsive forces may effectively act between the first to fourth magnetic members 1252. The attractive or repulsive force between the first magnetic part 1150 and the second magnetic part 1250 according to the rotation around the rotation shaft 1411 of the second rotating member 1420 may be prevented from operating ineffectively.

Specifically, as the second rotation member 1420 is moved according to a movement of the wing member 1430, the attractive force may effectively act between the first magnetic member 1151 and the third magnetic member 1251 and between the second magnetic member 1152 and the fourth magnetic member 1252 in the first state. Also, in the process of converting from the first state to the second state, the repulsive force may effectively act between the first magnetic member 1151 and the fourth magnetic member 1252 and between the second magnetic member 1152 and the third magnetic member 1251 according to the rotation around the rotation shaft 1411 of the second rotation member 1420.

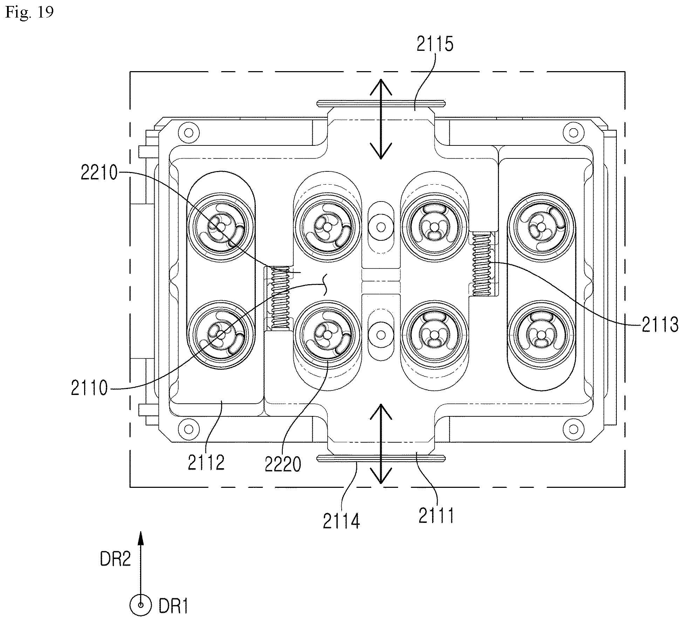

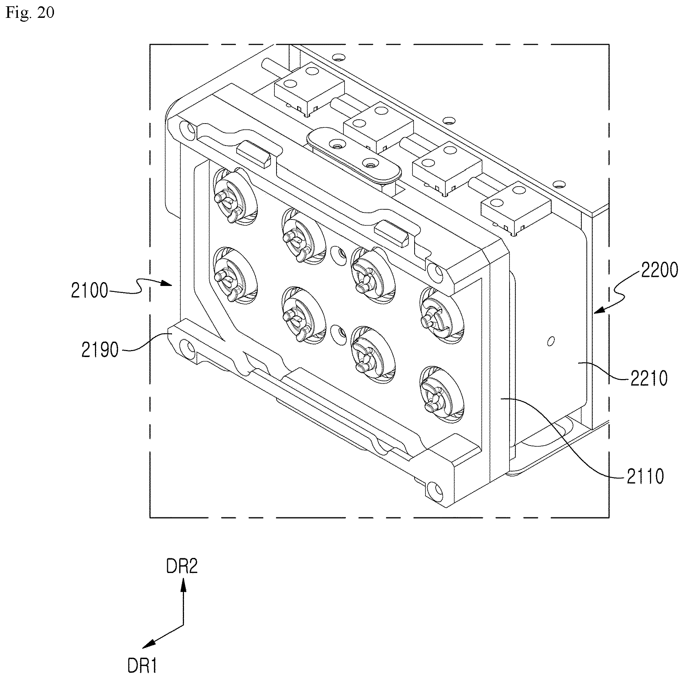

FIG. 18 is a perspective view illustrating a surgical instrument fixing device according to another embodiment of the present invention, FIG. 19 is a plan view illustrating the surgical instrument fixing device according to another embodiment of the present invention, and FIG. 20 is a perspective view illustrating a surgical instrument fixing device according to another embodiment of the present invention.

The same or similar components described above in FIGS. 2 to 14 are designated by the same name or reference numerals in which numbers in low place values are matched, and a description thereof will be omitted.

Referring to FIGS. 18 to 20, a first body 2100 may include a first body part 2110, and a second body 2200 may include a second body part 2220.

A first accommodating part 2120 may be defined in the first body part 2110, and a second accommodating part 2220 may be defined in the second body part 2210.

According to an embodiment of the present invention, the first accommodating part 2120 may be defined as an opening, and the second accommodating part 2220 may protrude from the first body part 2100 and be accommodated in the opening. The first body 2100 and the second body 2200 may be coupled through a coupling structure of the first accommodating part 2120 and the second accommodating part 2220.

The first body part 2110 may include a first frame 2111 and a second frame 2112. A first support 2114 may be defined in the first frame 2111, and a second support 2115 may be defined in the second frame 2112. The first frame 2111 and the second frame 2112 may be relatively moved in the second direction DR2 by an external force applied to the first support 2111 and/or the second support part.

According to an embodiment of the present invention, the first body 2100 may include a cover 2190 that covers the first body part 2110. An opening that accommodates the second accommodating part 2220 may be defined in the cover 2190.

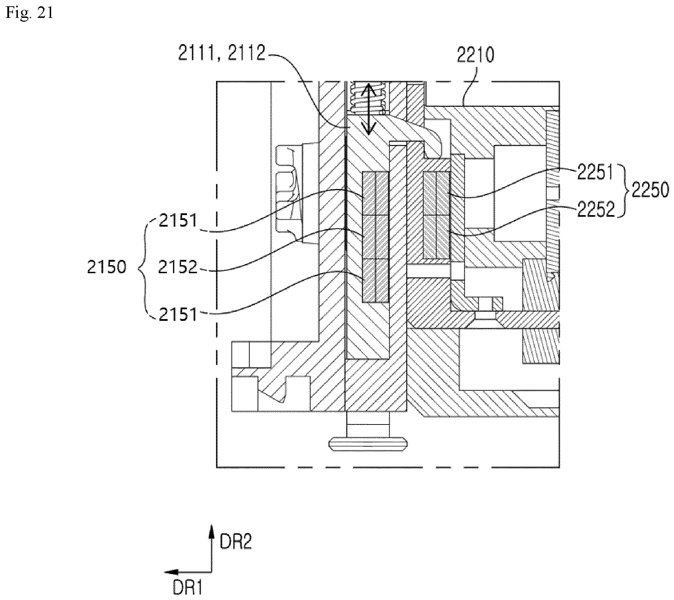

FIG. 21 is a schematic cross-sectional view illustrating the surgical instrument fixing device according to another embodiment of the present invention.

Referring to FIG. 21, the first magnetic part 2150 may be disposed on the first frame 2111 and/or the second frame 2112, and the second magnetic part 2250 may be disposed on the second body part 2210.

According to an embodiment of the present invention, each of the first magnetic part 2150 and the second magnetic part 2250 may include a plurality of magnetic members. The first magnetic part 2150 may include a plurality of first magnetic members 2151 and a plurality of second magnetic members 2152, and the first magnetic member 2151 and the second magnetic member 2152 may have different magnetism. The second magnetic part 2250 may include a plurality of third magnetic members 2251 and a plurality of fourth magnetic members 2252, and the third magnetic member 2251 and the fourth magnetic member 2252 may have different magnetism.

Here, the magnetic member may be a permanent magnet. However, the embodiment of the present invention is limited thereto. For example, the magnetic member may be an electromagnet, or a portion of the magnetic member may be a permanent magnet and a portion thereof is an electromagnet.

The first magnetic member 2151 and the third magnetic member 2251 may have different magnetism, and the second magnetic member 2152 and the fourth magnetic member 2252 may have different magnetism.

In the first state, the first magnetic member 2151 and the third magnetic member 2251 may overlap each other on the plane, and the second magnetic member 2152 and the fourth magnetic member 2252 may overlap each other on the plane. As an attractive force occurs between the first magnetic member 2151 and the third magnetic member 2251 and between the second magnetic member 2152 and the fourth magnetic member 2252, positions of the first body 2100 and the second body 2200 may be fixed.

In the process of converting from the first state to the second state, the first magnetic member 2151 and the fourth magnetic member 2252 may overlap each other on the plane, and the second magnetic member 2152 an the third magnetic member 2251 may overlap each other on the plane. A repulsive force may occur between the first magnetic member 2151 and the fourth magnetic member 2252 and between the second magnetic member 2152 and the third magnetic member 2251 to release the position fixed state of the first body 2100 and the second body 2200, and the first body 2100 and the second body 2200 may be spaced apart from each other.

In this case, the first magnetic member 2151 and the third magnetic member 2251 may partially overlap each other, and the second magnetic member 2152 and the fourth magnetic member 2252 may partially overlap each other. However, the embodiment of the present invention is not limited thereto.

According to an embodiment of the present invention, the first magnetic member 2151 and the second magnetic member 2152 may be arranged alternately in one direction, and the third magnetic member 2251 and the fourth magnetic member 2252 may be arranged alternately in one direction. Here, the one direction may be the second direction 2DR2 crossing the first direction.

According to the relative movement of the first frame 2111 and the second frame 2112, an overlapping relationship of the first to fourth magnetic members 2151, 2152, 2251, and 2252 may be changed. Specifically, in the first state, the first magnetic member 2151 and the third magnetic member 2251 may overlap each other, and the second magnetic member 2152 and the fourth magnetic member 2252 may overlap each other. As the first frame 2111 and the second frame 2112 are relatively moved in the second direction 2DR2 in the process of converting from the first state to the second state, the first magnetic member 2151 and the fourth magnetic member 2252 may overlap each other, and the second magnetic member 2152 and the third magnetic member 2251 may overlap each other.

A surgical device 5300 may include an arm module 5100 and a surgical instrument module 5200.

The arm module 5100 may include at least one of a base 5101, a guide tube 5104, a motor 5102, a first interface 5110, and a first coupler 5112.

The surgical instrument module 5200 may include at least one of a housing 5201, a second interface 5210, a second coupler 5230, an elongated body 510, and an end effector 501.

The arm module 5100 may maintain a position and an angle of the guide tube 5104 at a predetermined position with respect to an eye that is a subject of precision surgery. The arm module 5100 may include a plurality of operating arms having a parallelogram structure. The operating arms having the parallelogram structure may be more robust than a robot arm having a cantilever structure in terms of a position error, an angular error, a translational movement error, a rotational movement error, vibration, a backlash, hysteresis, and a nonlinear error. The plurality of operating arms may form a moving unit for the precise surgery.

While the robot arm having the cantilever structure is used for general surgery requiring moderate precision, the arm module 5100 according to the present invention may be used for surgery requiring high precision such as eye surgery of the present invention. When the arm module 5100 according to the present invention has, e.g., a rotational degree of freedom around x-axis, the plurality of arms having the parallelogram structure move to realize the corresponding degree of freedom. Thus, the plurality of arms may be more robust to various errors and improve surgical precision than the single arm such as a cantilever.

The guide tube 5104 may support the elongated body 510 of the surgical instrument module 5200 at a position adjacent to the eye that is the subject of the surgery. The elongated body 510 that is vulnerable to vibration or bending during high precision surgery may have both ends supported by the guide tube 5104 and the housing 5201. The elongated body 510 may have a support structure having a both ends supported beam shape in which both ends are supported by the guide tube 5104 of the arm module 5100 and the surgical instrument module 5200.

When the arm module 5100 is capable of performing rotational movement with two degrees of freedom and transitional movement with two degrees of freedom, the arm module 5100 may set the position and angle of the guide tube 5104 to a desired position and angle while performing the rotational movement with two degrees of freedom and the transitional movement with two degrees of freedom.

Various kinds of surgical instrument modules 5200 may be attached to and detached from the arm module 5100 for replacement depending on a surgical procedure. As a result, the elongated body 510 of the surgical instrument module 5200 may be attached to and detached from the guide tube 5104 that is set to the desired position and angle by the arm module 5100.

A coupling surface between the surgical instrument module 5200 and the arm module 5100 may be obtained by a method in which the first interface 5110 of the arm module 5100 and the second interface 5210 of the surgical instrument module 5200 are coupled while facing each other. The coupling surface between the surgical instrument module 5200 and the arm module 5100 may include the first interface 5110 of the arm module 5100 and the second interface 5210 of the surgical instrument module 5200, which are coupled to each other.

The coupling surface between the surgical instrument module 5200 and the arm module 5100 or between the first interface 5110 and the second interface 5210 may correspond to a sterilization interface. Based on the sterilization interface, a portion on which the arm module 5100 is disposed may correspond to a sterilized portion and be wrapped by sterilization vinyl 6000 for repeated use. Based on the sterilization interface, a portion on which the surgical instrument module 5200 is disposed may correspond to a non-sterilized portion and be discarded after being used a certain number of times.

The surgical instrument module 5200 may be discarded after being used a certain number of times because the surgical instrument module is only sterilized to a predetermined level and exposed to the same space as the subject of the surgery.

Thus, it is recommended that only the minimum number of components are mounted to the surgical instrument module 5200 that is a disposable portion. The surgical instrument module 5200 may include the housing 5201, the wire, a pulley around which the wire is wound, and the second coupler 5230 connected to the pulley. Expensive components such as the motor 5102 and a motor control unit may be installed on the arm module 5100 that is the sterilized portion.

The motors 5102 may be arranged annularly or with equal intervals around a first hole 5111 or a guide tube hole 5105 for vibration reduction or mass distribution.

The pulley around which the wire is wound is also preferably installed on the arm module 5100. In this case, however, since the coupling surface between the first interface 5110 and the second interface 5210 inevitably has a structure in which the wire is coupled and separated, this structure may not be substantially realized. Thus, inevitably, the first interface 5110 and the second interface 5210 have a structure in which the first coupler 5112 and the second coupler 5230 are attached to and detached from each other, and the discarded surgical instrument module 5200 has a structure in which the pulley and the wire, as the minimum number of components, are installed in the housing 5201.

When the surgical instrument module 5200 is mounted to the arm module 5100, the surgical instrument module 5200 may move from a distal direction that is a direction away from the subject of the surgery to a proximal direction that is a direction adjacent to the subject of the surgery.

When the second interface 5210 of the surgical instrument module 5200 is coupled to the first interface 5110 of the arm module 5100, the surgical instrument module 5200 may be completely mounted. When the surgical instrument module 5200 is coupled, the elongated body and the end effector 501 may sequentially pass through the first hole 5111 of the first interface 5110 and the guide tube hole 5105 of the guide tube 5104. The motor 5102 of the arm module 5100 may be disposed between the first hole 5111 and the guide tube hole 5105. The first hole 5111, the guide tube hole 5105, and the arm module 5100 may correspond to the sterilized portion that is used repeatedly instead of being discarded and be wrapped by sterilization vinyl 6000. An opened portion of the sterilization vinyl 6000 may be the first hole 5111 and the guide tube hole 5105, to which the elongated body is mounted. A portion except for the first hole 5111 and the guide tube hole 5105 may be sealed by the sterilization vinyl 6000.

When the surgical instrument module 5200 is separated from the arm module 5100, the second interface 5210 of the surgical instrument module 5200 may be separated from the first interface 5110 of the arm module 5100. The surgical instrument module 5200 may be completely separated when the surgical instrument module 5200 moves from the proximal direction to the distal direction. When the surgical instrument module 5200 is separated, the elongated body and the end effector 501 may sequentially pass through the guide tube hole 5105 and the first hole 5111 of the first interface 5110. The motor 5102 of the arm module 5100 may be disposed between the first hole 5111 and the guide tube hole 5105. Various kinds of surgical instrument modules 5200 may be coupled or separated during the surgical procedure. The surgical instrument module 5200 that is repeatedly used may be discarded and replaced with the new surgical instrument module 5200 to prevent infection of a patient. To this end, the first hole 5111 and the guide tube hole 5105 may be defined.

The arm module 5100 may have a structure in which the guide tube hole 5105, the motor 5102, and the first hole 5111 of the first interface 5110 are sequentially arranged in a direction from a proximal portion to a distal portion.

The surgical instrument module 5200 may have a structure in which the end effector 501, the elongated module, the second interface 5210, and the housing 5201 are sequentially arranged in the direction from the proximal portion to the distal portion.

The first interface 5110 may include the first coupler 5112 connected to the motor 5102. The second interface 5210 may include the second coupler 5230 connected to the wire or pulley in the housing 5201. A torque of the motor 5102 may be transmitted to the pulley in the housing 5201 by the first coupler 5112 and the second coupler 5230 that are coupled in an axial direction. Rotation of the pulley may be converted into tension of the wire. The wire extending into the elongated body 510 may use the tension to operate the end effector 501.

Since the first interface 5110 and the second interface 5210 form the coupling surface, the housing 5201 of the surgical instrument module 5200 may be exposed to the non-sterilized portion or the same space as the subject of the surgery.

The surgical instrument fixing device according to the embodiment of the present invention may align the surgical instrument to the exact position and fix the surgical instrument to prevent the surgical instrument from being deviated from the aligned position.

The surgical instrument fixing device according to the embodiment of the present invention may firmly couple the bodies that fix the surgical instrument to each other.

The surgical instrument fixing device according to the embodiment of the present invention may allow the bodies that fix the surgical instrument to be easily coupled to each other.

The surgical instrument fixing device according to the embodiment of the present invention may easily eliminate the error generated when the bodies that fix the surgical instrument are coupled to each other.

Although the exemplary embodiments of the present invention have been described, it is understood that the present invention should not be limited to these exemplary embodiments but various changes and modifications can be made by one ordinary skilled in the art within the spirit and scope of the present invention as hereinafter claimed. Therefore, the detailed description of the present invention does not intend to limit the present invention to the disclosed embodiments, and the technical scope of the present invention should be defined by the following claims.

### [Description of the reference numerals]

10: Surgical instrument
100: First body
110: First body part
120: First accommodating part
130: First fastening part
200: Second body
210: Second body part
220: Second accommodating part
230: Second fastening part
300: Drive unit
5100: Arm module
5101: Base
5102: Motor
5104: Guide tube
5105: Guide tube hole
5110: First interface
5111: First hole
5112: First coupler
5200: Surgical instrument module
501: End effector
510: Elongated body
5201: Housing
5210: Second interface
5230: Second coupler
5300: Surgical device
6000: Sterilization vinyl

## Claims

1. A surgical device comprising:
a first body having a first fastening part; and
a second body coupleable to the first body and having a second fastening part,
wherein, when the first body and the second body are coupled, the first fastening part is accommodated in the second fastening part; and
the first body further comprises a first body part and a first accommodating part configured to accommodate a surgical instrument,
the first fastening part comprises a plurality of protrusions extending from a surface of the first body part,
the second body comprises a second body part and a second accommodating part for receiving the surgical instrument,
the second fastening part comprises a plurality of grooves recessed from a surface of the second body; and
the plurality of protrusions are circumferentially arranged on the first body part such that an angle between adjacent protrusions of the plurality of protrusions remains approximately equal, and
wherein the plurality of grooves are circumferentially arranged on the second body part such that an angle between adjacent protrusions of the plurality of grooves remains approximately equal.

2. The surgical device of claim 1, wherein the each of the plurality of protrusions comprises a first protruding portion and a second protruding portion, and
the first protruding portion is disposed proximate the surface of the first body part and the second protruding portion is spaced from the surface of the first body part,
wherein at least a portion of the second protruding portion is curved, and
a cross-sectional area of the plurality of protrusions decreases in a direction from the second protruding portion to the first protruding portion.

3. The surgical device of claim 1, wherein the first fastening part further comprises a first support configured to support the protrusions and a second support configured to support the first support on the first body, and
wherein the second support further comprises a spring member configured to elastically support the first support.

4. A surgical device comprising:
a first body to which a surgical instrument is fixed, the first body comprising:
a first body part having a first accommodating part for receiving the surgical instrument;
a first magnetic part; and
a first coupling part disposed in the first body part, and
a second body, the second body comprising:
a second body part having a second accommodating part for receiving the surgical instrument is accommodated;
a second magnetic part and generating a repulsive force or attractive force between the first magnetic part and the second magnetic part; and
a second coupling part coupled to the first coupling part,
wherein, in a first state, the first coupling part and the second coupling part are coupled, and an attractive force is generated between the first magnetic part and the second magnetic part to fix the first body and the second body, and
in a second state, a repulsive force is generated between the first magnetic part and the second magnetic part to release a fixed state of the first body and the second body, and release a coupled state of the first coupling part and the second coupling part.

5. The surgical device of claim 4, wherein the first body further comprises a ratchet, the ratchet comprising:
a first part protruding from the first body toward the second body, and
a second part bent from the first part,
wherein the second coupling part comprises a ratchet groove capable of accommodating the ratchet and surgical instrument fixing device having a step configured to support the second part accommodated in the ratchet groove.

6. The surgical device of claim 4, wherein the first magnetic part further comprises a plurality of first magnetic members and a plurality of second magnetic members,
the second magnetic part comprises a plurality of third magnetic members having a same magnetism as the second magnetic members and a plurality of fourth magnetic members having a same magnetism as the first magnetic members,
in a first state, the first magnetic member and the third magnetic member overlap on a plane, and the second magnetic member and the fourth magnetic member overlap on a plane,
when transitioning from the first state to the second state, first magnetic member is flat with the fourth magnetic member of the relative movement of the first frame and/or the second frame in the second direction, and
the second magnetic member overlaps the third magnetic member on a plane.

7. The surgical device of claim 4, wherein the first body comprises a first inclined portion having a first inclined surface inclined in a direction crossing the first direction,
the second coupling part comprises a second inclined portion inclined in a direction crossing the first direction at a position corresponding to the first inclined portion and having a second inclined surface facing the first inclined surface, and
when transitioning from the first state to the second state, the second inclined surface moves along the first inclined surface.

8. The surgical device of claim 4, wherein the first body further comprising a position adjustment unit for moving the position of the first magnetic part.

9. A surgical device comprising:
an arm module having a guide tube; and
a surgical instrument module having an end effector configured to act on a subject of surgery, an elongated body to which the end effector is mounted, and a housing to which the elongated body is mounted,
wherein a first interface of the arm module and a second interface of the surgical instrument module form a coupling surface, and
the surgical instrument module is attached and detached through the coupling surface.

10. The surgical device of claim 9, wherein the elongated body has a portion supported by the guide tube of the arm module and the other end supported by the housing of the surgical instrument module.

11. The surgical device of claim 9, wherein a driving force of the end effector of the surgical instrument module is provided from a drive source of the arm module.

12. The surgical device of claim 11, wherein, when the surgical instrument module is coupled, the elongated body and the end effector sequentially pass through a first hole of the first interface of the arm module and a guide tube hole of the guide tube, the drive source of the arm module is disposed between the first hole and the guide tube hole, and the drive source provides the driving force of the end effector of the surgical instrument module.

13. The surgical device of claim 12, wherein a first hole of the first interface of the arm module and a guide tube hole of the guide tube are defined, and
the first hole, the guide tube hole, and the arm module correspond to a sterilized portion that is repeatedly used and wrapped by sterilization vinyl.

14. The surgical device of claim 9, wherein, when the surgical instrument module is separated from the arm module, the second interface of the surgical instrument module is separated from the first interface of the arm module, and
when the surgical instrument module moves from a proximal direction to a distal direction, the surgical instrument module is completely separated.
